(19) Europäisches Patentamt
European Patent Office
Office européen des brevets

(11) EP 3 017 819 B1

(12) **EUROPEAN PATENT SPECIFICATION**

(45) Date of publication and mention of the grant of the patent:
**29.08.2018 Bulletin 2018/35**

(21) Application number: **14819540.7**

(22) Date of filing: **02.07.2014**

(51) Int Cl.:
***A61K 31/704*** (2006.01) ***A61K 31/70*** (2006.01)
***A61P 25/28*** (2006.01) ***A61P 25/00*** (2006.01)
***A61K 36/28*** (2006.01)

(86) International application number:
**PCT/KR2014/005884**

(87) International publication number:
**WO 2015/002450 (08.01.2015 Gazette 2015/01)**

(54) **PHARMACEUTICAL COMPOSITION FOR PREVENTING OR TREATING COGNITIVE IMPAIRMENT OR CONCENTRATION IMPAIRMENT DISORDERS, COMPRISING ECLALBASAPONIN OR DERIVATIVE THEREOF**

PHARMAZEUTISCHE ZUSAMMENSETZUNG ZUR PRÄVENTION ODER BEHANDLUNG VON KOGNITIVER BEEINTRÄCHTIGUNG ODER KONZENTRATIONSSTÖRUNGEN MIT ECLALBASAPONIN ODER DERIVAT DAVON

COMPOSITION PHARMACEUTIQUE POUR LA PRÉVENTION OU LE TRAITEMENT DES TROUBLES LIÉS À LA DÉFICIENCE COGNITIVE OU À LA DIMINUTION DE LA CONCENTRATION COMPRENANT DE L'ECLALBASAPONINE OU SES DÉRIVÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.07.2013 KR 20130077882**

(43) Date of publication of application:
**11.05.2016 Bulletin 2016/19**

(73) Proprietor: **University-Industry Cooperation Group of Kyung Hee University**
**Gyeonggi-do 446-701 (KR)**

(72) Inventors:
- **RYU, Jong Hoon**
  **Seoul 135-280 (KR)**
- **CHEONG, Jae Hoon**
  **Namyangju-si**
  **Gyeonggi-do 472-743 (KR)**
- **SHIN, Chan Young**
  **Seoul 158-756 (KR)**
- **JANG, Dae Sik**
  **Seoul 136-726 (KR)**
- **AHN, Young Jae**
  **Gyeongju-si**
  **Gyeongsangbuk-do 780-702 (KR)**
- **KIM, Ha Young**
  **Gunwi-gun**
  **Gyeongsangbuk-do 716-801 (KR)**

(74) Representative: **D Young & Co LLP**
**120 Holborn**
**London EC1N 2DY (GB)**

(56) References cited:
**WO-A2-2004/069182**
**JP-A- 2000 212 047**
**KR-A- 20020 059 906**
**KR-A- 20100 003 045**
**KR-A- 20130 060 835**
**US-A1- 2010 190 968**
**US-A1- 2012 321 694**

- **SHOJI YAHARA ET AL: "Studies on the Constituents of Compositae Plants. Part II. Oleanane Glycosides from Eclipta alba.", CHEMICAL AND PHARMACEUTICAL BULLETIN, vol. 42, no. 6, 1 January 1994 (1994-01-01), pages 1336-1338, XP055323103, JP ISSN: 0009-2363, DOI: 10.1248/cpb.42.1336**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- **YUKO NAKAHARA ET AL: "Oleanene Glycosides of the Aerial Parts and Seeds of Bupleurum falcatum and the Aerial Parts of Bupleurum rotundifolium, and Their Evaluation as Anti-hepatitis Agents", CHEMICAL AND PHARMACEUTICAL BULLETIN, vol. 59, no. 11, 1 November 2011 (2011-11-01), pages 1329-1339, XP055323021, JP ISSN: 0009-2363, DOI: 10.1248/cpb.59.1329**
- **ERIC K. CHEA ET AL: "Synthesis and Preclinical Evaluation of QS-21 Variants Leading to Simplified Vaccine Adjuvants and Mechanistic Probes", JOURNAL OF THE AMERICAN CHEMICAL SOCIETY, vol. 134, no. 32, 15 August 2012 (2012-08-15), pages 13448-13457, XP055239148, US ISSN: 0002-7863, DOI: 10.1021/ja305121q**
- **RAHMAN, M. S. ET AL.: 'Antimicrobial activity and cytotoxicity of Eclipta prostrata' ORIENTAL PHARMACY AND EXPERIMENTAL MEDICINE vol. 8, no. 1, 2008, pages 47 - 52, XP053006378**

## Description

[Technical Field]

The present disclosure relates to a pharmaceutical composition for use in preventing or treating cognitive impairment or concentration impairment comprising Eclalbasaponin or derivative thereof.

Also, the present disclosure relates to a food composition for use in preventing or improving cognitive impairment or concentration impairment comprising Eclalbasaponin or derivative thereof.

[Background]

Average human life span was extended nearly twice over the past half century due to development of medical technology and improvement of the living standards, and thereby the proportion of the elderly population to total population has increased rapidly. As society is gradually aging, senile dementia has emerged as the largest health problem for human being to be solved in the 21st century. Accordingly, there is a growing demand for the development of functional materials and foods for preventing and treating cognitive disorders including dementia.

Dementia, a representative disease of cognitive disorders, is a morbid phenomenon that should be distinguished from normal aging. Dementia is categorized into Alzheimer's dementia, vascular dementia, dementia caused by alcoholism, dementia caused by external injury and dementia comes after Parkinson's disease as a sequel depending on its causes. It is known that Alzheimer's disease accounts for 50% to 70% of diseases causing dementia and vascular dementia is known to be the second most frequent cause of dementia.

A research paper reported that Alzheimer's dementia is a sort of chronic neurodegenerative disease showing higher cerebral dysfunction such as loss of memory, obscure consciousness, space-time confusion and disorders of thinking, calculating, judgment and common sense. Alzheimer's dementia is known as the most common form of dementia in the elderly population. There is a case of Alzheimer's dementia occurrence at relatively young age, and occurrence ratio increases twice in every 5 years in the age range of 65 to 85. An onset mechanism of Alzheimer's dementia is unclear, but decrease of acetylcholine function in the central nervous system is the most common phenomenon. Therefore, administration of acetylcholine precursors or drugs inhibiting decomposition of acetylcholine for increasing concentration of acetylcholine in brain has been used to treat Alzheimer's dementia. Thus, single use of acetylcholine esterase (hereinafter, 'AChE') inhibitors or combined use with previous cholinesterase inhibitors is used as a medicament for Alzheimer's dementia. Representative drugs would be tacrine, donepezil, rivastigmine, galantamine and the like. All of these merely delay the progression of disease and are not that effective in direct treatment. Also, the therapeutic range of these is limited to the initial stage, therefore the efforts to develop a drug for treating the root cause of Alzheimer's dementia have been made (Terry and Buccafusco, 2003; Kar *et al.,* 2004; Akhondzadeh *et al.,* 2008; Cummings *et al.,* 2008; Voss *et al.,* 2008).

Meanwhile, vascular dementia is mostly caused by damages to the brain cells due to insufficient blood supply to many areas of the brain by cerebrovascular atherosclerosis. Reasons for onset of vascular dementia and Alzheimer's dementia are different, but it eventually impairs the memory in common.

As explained, brain function decline and concentration decrease are caused by being aged, and by external factors such as stress and the like.

Shoji Yahara et al., Chem. Pharm. Bull. 42(6), 1336 - 1338 (1994) describes the isolation and characterisation of eclalbasaponins I-VI from whole parts of *Eclipta alba* Hassk. Yuko Nakahara et al., Chem. Pharm. Bul. 59(11), 1329 - 1339 (2011) describes the isolation and characterisation of Eclalbasaponin derivatives from the aerial parts of *Bapleurum falcatum and Bapleurum rotundifolium*.

WO 2004/069182 describes methods useful for effecting prophylaxis or treatment of Alzeimer's disease.

US 2012/0321694 describes compositions and methods that are useful for preventing and treating Alzheimer's disease.

Eric K. Shea et al., J. Am. Chem. Soc., 134, 13448 - 13457 (2012), describes the design, synthesis and preclinical evaluation of a modified saponin immunoadjuvants.

The present inventors discovered a crude extract and its active ingredient that effectively enhance memory and learning ability, and improve concentration ability while researching on development of an effective medicament for preventing or treating cognitive disorders such as dementia, and improving concentration ability, and accordingly completed the present disclosure.

[Summary]

[Technical Problem]

It is an object of the present disclosure to provide a pharmaceutical composition for use in preventing or treating cognitive impairment or concentration impairment, comprising Eclalbasaponin or derivative thereof, represented by Formula 1 below, as an active ingredient:

[Formula 1]

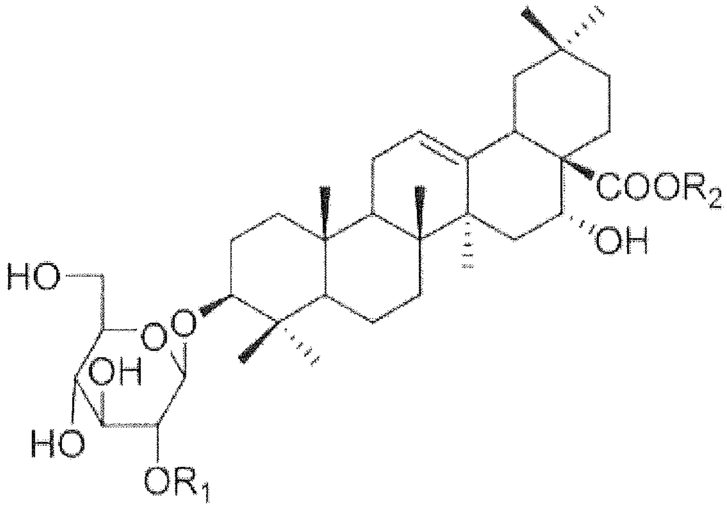

In Formula 1,

$R_1$ is H, $SO_3H$ or glucose (Glc), and
$R_2$ is H or glucose (Glc)

Another object of the present disclosure is to provide a pharmaceutical composition for use in preventing or treating cognitive impairment or concentration impairment, comprising *Eclipta prostrata* extract containing Eclalbasaponin or derivative thereof, represented by Formula 1 above.

Still another object of the present disclosure is to provide a food composition for preventing or ameliorating cognitive impairment or concentration impairment, comprising Eclalbasaponin or derivative thereof, represented by Formula 1 above.

Still another object of the present disclosure is to provide a food composition for use in preventing or ameliorating cognitive impairment or concentration impairment, comprising *Eclipta prostrata* extract containing Eclalbasaponin or derivative thereof, represented by Formula 1 above.

[Solution to Problem]

**Pharmaceutical Composition**

Composition comprising Eclalbasaponin

The present disclosure provides the pharmaceutical composition for use in preventing or treating cognitive impairment or concentration impairment, comprising Eclalbasaponin or derivative thereof, represented by Formula 1 below, as an active ingredient:

[Formula 1]

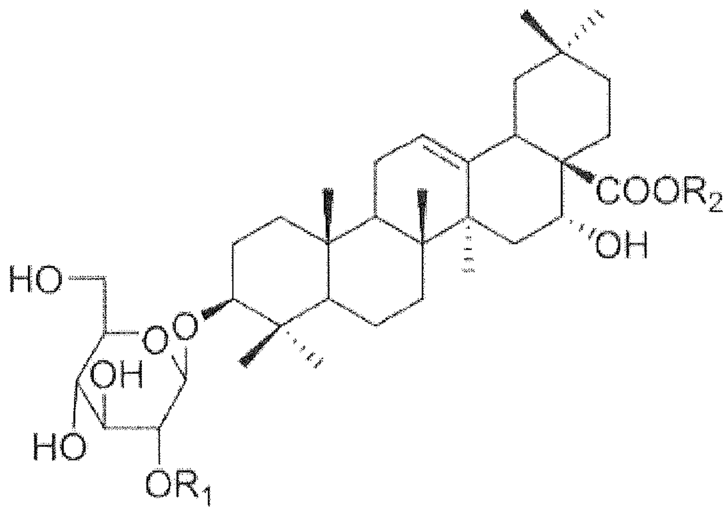

in Formula 1,

$R_1$ is H, $SO_3H$ or glucose (Glc), and
$R_2$ is H or glucose (Glc).

In some embodiment of the present disclosure, the Eclalbasaponin or derivative thereof can be selected from among Eclalbasaponin I to VI listed in Table 1 below. Preferably, it can be Eclalbasaponin I or II, and most preferably, it can be Eclalbasaponin II.

[Table 1]

| Compound | $R_1$ | $R_2$ |
|---|---|---|
| Eclalbasaponin I | H | Glc |
| Eclalbasaponin II | H | H |
| Eclalbasaponin III | Glc | Glc |
| Eclalbasaponin IV | Glc | H |
| Eclalbasaponin V | $SO_3H$ | H |
| Eclalbasaponin VI | $SO_3H$ | Glc |

The Eclalbasaponin or derivative thereof can be yielded from *Eclipta prostrata,* preferably it can be yielded from a whole plant of *Eclipta prostrata.*

In detail, the Eclalbasaponin or derivative thereof can be yielded from an *Eclipta prostrata* extract. An extraction solvent for the *Eclipta prostrata* extract can be water, alcohol, hexane or mixtures thereof. It is preferable to use $C_1$-$C_4$ lower alcohol for the alcohol, and it is most preferable to use methanol or ethanol. Shaking extraction, Soxhlet extraction or reflux extraction can be used as an extraction method but not limited to these. It is preferable for extraction temperature to be 40-100 °C, and it is most preferable for the temperature to be 60-80 °C. Also, it is preferable for extraction duration to be 2-24 hours, and extraction times to be 1-5 times.

Also, the Eclalbasaponin or derivative thereof can be yielded from a fraction of water or alcohol extract of *Eclipta prostrata.* For instance, the fraction can be hexane, ethyl acetate, butanol and water fractions of the water or alcohol extract of *Eclipta prostrata,* and preferably, it can be ethyl acetate fraction of ethanol extract of *Eclipta prostrata.*

Additionally, the Eclalbasaponin or derivative thereof can be used by purchasing from a market or be yielded by organic synthesis.

The pharmaceutical composition of the present disclosure comprising Eclalbasaponin or derivative thereof can be used for preventing or treating cognitive impairment or concentration impairment.

The cognitive impairment of the present disclosure can be all kinds of diseases caused by impairments of memory, space-time cognition, judging ability, executive function, linguistic ability and the like such as delirium, dementia or amnesia. Also, the dementia can be occurred by various causes such as Alzheimer's dementia, vascular dementia, dementia caused by alcoholism, dementia caused by external injury or dementia comes after Parkinson's disease as a sequel. Preferably, the dementia can be Alzheimer's dementia or vascular dementia.

In detail, the pharmaceutical composition comprising Eclalbasaponin or derivative thereof shows remarkable effect in improving learning ability, space-time cognition and memory to a high level in an animal model of memory impairment that is induced by scopolamine. Therefore, it is confirmed that the pharmaceutical composition exhibits superior activity in preventing or treating cognitive impairment or concentration impairment.

In addition, the pharmaceutical composition comprising Eclalbasaponin or derivative thereof of the present disclosure can inhibit weakening of memory in the scopolamine-induced mouse with a small dose, and can improve concentration ability.

Furthermore, the Eclalbasaponin or derivative thereof is an ingredient included in the herbal drug, and thus it can effectively treat cognitive disorder and improve concentration ability without side effects.

Accordingly, the pharmaceutical composition of the present disclosure can be useful for preventing or treating cognitive disorders, especially delirium, dementia or amnesia.

It is preferable for the Eclalbasaponin or derivative thereof of the present disclosure to be included 0.1-50.0 wt% based on the total weight of the pharmaceutical composition of the present disclosure. However, the content is not limited to the above, and can vary depending on status of patients, and type and progression of the disease.

Apropos of the pharmaceutical composition of the present disclosure comprising Eclalbasaponin or derivative thereof for use in preventing or treating cognitive or concentration impairment, the Eclalbasaponin or derivative thereof can be administered once or several times at a dose of about 1 mg through 1 g per a day as for
the adults, and preferably, the administration can be once or several times at a dose of about 30 mg through 120 mg. However, the dosage of the Eclalbasaponin or derivative thereof can be properly adjusted depending on patient's status such as severity, age, sex, weight and the like, types of drug formulation, route of administration and duration of administration.

Composition comprising *Eclipta prostrata* extract

Moreover, the present disclosure provides the pharmaceutical composition for use in preventing or treating cognitive impairment or concentration impairment, comprising *Eclipta prostrata* extract containing Eclalbasaponin or derivative thereof, represented by Formula 1 below as an active ingredient.

[Formula 1]

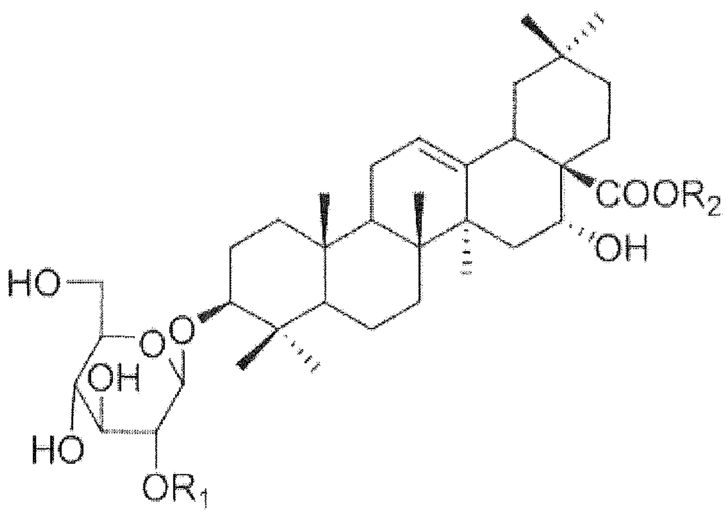

In Formula 1,

$R_1$ is H, $SO_3H$ or glucose (Glc), and
$R_2$ is H or glucose (Glc)

The *Eclipta prostrata* extract comprises Eclalbasaponin or derivative thereof, and likewise, the pharmaceutical composition comprising the *Eclipta prostrata* extract as an active ingredient exhibits superior activity in preventing or treating cognitive impairments, and improves concentration ability because it shows remarkable effect in improving learning ability, space-time cognition, memory and concentration ability to a high level in an animal model of memory impairment that is induced by scopolamine. Also, it can prevent or treat cognitive impairment or concentration impairment without side effects because it is an herbal extract.

Therefore, the pharmaceutical composition comprising the *Eclipta prostrata* extract as an active ingredient can be useful for preventing or treating cognitive impariments, especially delirium, dementia or amnesia.

The pharmaceutical composition of the present disclosure can include 0.1-50.0 wt% of the *Eclipta prostrata* extract of the present disclosure based on the total weight of the composition. However, the content is not limited to the above, and can vary depending on status of patient, and type and progression of the disease.

Apropos of the pharmaceutical composition of the present disclosure comprising the *Eclipta prostrata* extract for preventing or treating cognitive or concentration impairment, the *Eclipta prostrata* extract can be administered once or several times at a dose of about 10 mg through 2 g per a day as for the adults, and preferably, the administration can be once or several times at a dose of about 300 mg through 1200 mg. However, the dosage of the *Eclipta prostrata* extract can be properly adjusted depending on patient's status such as severity, age, sex, weight and the like, types of drug formulation, route of administration and duration of administration.

The pharmaceutical composition comprising Eclalbasaponin or derivative thereof, or the *Eclipta prostrata* extract containing the same as an active ingredient is safe for a long-term administration for preventing or treating cognitive disorders because it has no toxicity and side effects.

The pharmaceutical composition for use in preventing or treating cognitive impairment or concentration impairment comprising Eclalbasaponin or derivative thereof, or the *Eclipta prostrata* extract containing the same can comprise additives such as pharmaceutically acceptable diluents, binders, disintegrants, lubricants, pH modifiers, antioxidants, solubilizing agents and the like within the scope of the present disclosure.

The diluents can be sugar, starch, microcrystalline cellulose, lactose (lactose hydrate), glucose, D-mannitol, alginate, alkaline earth metal salt, clay, polyethylene glycol, anhydrous calcium hydrogen phosphate or mixtures thereof, and the like.

The binders can be starch, microcrystalline cellulose, highly dispersible silica, mannitol, D-mannitol, sucrose, lactose hydrate, polyethylene glycol, polyvinylpyrrolidone (povidone), polyvinylpyrrolidone copolymers (copovidone), hypromellose, hydroxypropyl cellulose, natural gum, synthetic gum, copovidone, gelatin or mixtures thereof and the like.

The disintegrants can be starch or modified starch such as sodium starch glycolate, corn starch, potato starch or pre-gelatinized starch and the like; clay such as bentonite, montmorillonite or veegum and the like; celluloses such as microcrystalline cellulose, hydroxypropyl cellulose or carboxymethyl cellulose and the like; algins such as sodium alginate or alginic acid and the like; cross-linked cellulose such as sodium croscarmellose; gums such as guar gum and xanthan gum and the like; cross-linked polymers such as cross-linked polyvinylpyrrolidone (crospovidone) and the like; effervescent agents such as sodium bicarbonate, citric acid and the like, or mixtures thereof.

The lubricants can be talc, stearic acid, magnesium stearate, calcium stearate, sodium lauryl sulfate, hydrogenated vegetable oil, sodium benzoate, sodium stearyl fumarate, glyceryl behenate, glyceryl monooleate, glyceryl monostearate, glyceryl palmitostearate, colloidal silicon dioxide, or mixtures thereof, and the like.

The pH modifiers can be acidifying agents and the like such as acetic acid, adipic acid, ascorbic acid, sodium ascorbate, sodium etherate, malic acid, succinic acid, tartaric acid, fumaric acid and citric acid; and alkalizing agents such as precipitated calcium carbonate, ammonia water, meglumine, sodium carbonate, magnesium oxide, magnesium carbonate, sodium citrate and tribasic calcium phosphate.

The antioxidants can be dibutyl hydroxy toluene, butylated hydroxyanisole, tocopherol acetate, tocopherol, propyl gallate, sodium hydrogen sulfite and sodium pyrosulfite and the like.

The solubilizing agent can be polyoxyethylene sorbitan fatty acid esters such as sodium lauryl sulfate and polysorbate and the like, sodium docusate, poloxamer and the like.

The pharmaceutical composition for use in preventing or treating cognitive impairment or concentration impairment comprising Eclalbasaponin or derivative thereof, or the *Eclipta prostrata* extract containing the same can be formulated as a solid preparation such as tablet, pill, powder, granule, capsule and the like for oral administration. These solid preparations can be prepared by mixing the herbal extract containing Eclalbasaponin with at least one excipient such as starch, calcium carbonate, sucrose or lactose, gelatin and the like. Also, lubricants such as magnesium stearate, talc and the like can be used together other than simple excipients. Furthermore, the pharmaceutical composition can be formulated as a liquid preparation such as suspension, liquid for internal use, emulsion, syrup and the like for oral administration. Various excipients such as humectant, sweetening agent, flavoring agent, preservative and the like other than water and liquid paraffin can be used for formulation of the liquid preparation.

Additionally, the pharmaceutical composition for use in preventing or treating cognitive impairment or concentration impairment comprising Eclalbasaponin or derivative thereof, or the *Eclipta prostrata* extract containing the same

can comprise sterile aqueous solution, nonaqueous solvent, suspension, emulsion, lyophilized formulation and suppository when formulating for parenteral administration.

The nonaqueous solvent and suspension can be propylene glycol, polyethylene glycol, vegetable oils such as olive oil, or injectable esters such as ethyl oleate. A base material of the suppository can be witepsol, macrogol, tween 61, cacao butter, laurinum, glycerogelatin and the like.

The term "administration" used in the present disclosure means introduction of the pharmaceutical composition of the present disclosure for preventing or treating cognitive disorders to patients *via* proper methods, and the route of administration of the pharmaceutical composition of the present disclosure for preventing or treating cognitive impairment or concentration impairment can be any general routes as long as it reaches to the targeted tissue. The administration route can be oral, intraperitoneal, intravenous, intramuscular, subcutaneous, intranasal, intrapulmonary, intrarectal, intracavitary, intraperitoneal and intrathecal administrations, but not limited to these. For instance, the pharmaceutical composition can be administered through oral, rectal or venous, muscular, subcutaneous, endometrial, or intracerebroventricular injection.

The pharmaceutical composition of the present disclosure for use in preventing or treating cognitive impairment or concentration impairment comprising Eclalbasaponin or derivative thereof, or the *Eclipta prostrata* extract containing the same can be administered once a day or divided into several times at regular intervals.

In addition, the pharmaceutical composition of the present disclosure for use in preventing or treating cognitive impairment or concentration impairment comprising Eclalbasaponin or derivative thereof, or the *Eclipta prostrata* extract containing the same can further comprise other active ingredients having effect on treating cognitive impairment or concentration impairment.

The pharmaceutical composition of the present disclosure comprising Eclalbasaponin or derivative thereof, or the *Eclipta prostrata* extract containing the same can be used solely or jointly with various treatment methods such as hormone therapy, drug therapy and the like to prevent or treat cognitive impairment or concentration impairment.

## Food Composition

The present disclosure provides the food composition represented by Formula 1 for use in preventing or ameliorating cognitive impairment or concentration impairment comprising Eclalbasaponin or derivative thereof, represented by Formula 1 below:

[Formula 1]

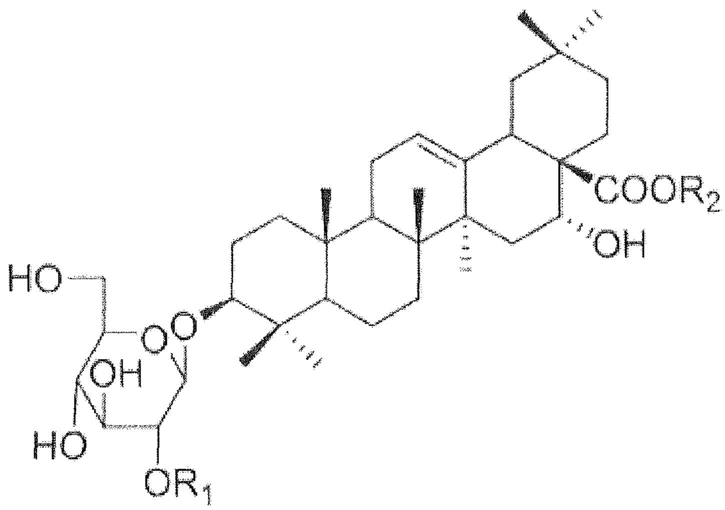

In Formula 1,

$R_1$ is H, $SO_3H$ or glucose (Glc), and
$R_2$ is H or glucose (Glc)

Also, the present disclosure provides the food composition for use in preventing or ameliorating cognitive impairment or concentration impairment comprising the *Eclipta prostrata* extract containing Eclalbasaponin or derivative

thereof, represented by Formula 1 below.

The food compositions of the present disclosure can be prepared by intactly adding Eclalbasaponin or derivative thereof, or the *Eclipta prostrata* extract containing the same thereto, or can be employed with additives and the like in other food compositions, a functional health food or a beverage.

For instance, the food composition of the present disclosure can comprise sweetening agents such as sucrose, granulated fructose, glucose, D-sorbitol, mannitol, isomalto oligosaccharide, stevioside, aspartame, potassium acesulfame, sucralose, and the like, acidifiers such as anhydrous citric acid, DL-malic acid, succinic acid and its salt, and the like, preservatives such as benzoic acid and its derivative, and the like, various nutritional supplements, vitamins, minerals (electrolyte), flavoring agents such as synthetic and natural flavoring agents; coloring agents; enhancer (cheese, chocolate and the like); pectic acid and its salt, alginic acid and its salt, organic acid, protective colloid thickener, pH modifier, stabilizer, preservative, glycerin, alcohol, carbonator used for carbonated beverage and the like. Also, the food composition of the present disclosure can comprise pulp for preparation of natural fruit and vegetable juice. The ratio of these additives can be decided within the range of about 20 parts of weight or below per 100 parts of weight of the food composition of the present disclosure.

When the food composition of the present disclosure is prepared as a beverage, it can further comprise flavoring agents or natural carbohydrate, which is commonly added to beverages. The natural carbohydrate can be monosaccharide such as glucose and fructose; disaccharide maltose such as maltose and sucrose; polysaccharide such as dextrin and cyclodextrin; or sugar alcohol such as xylitol, sorbitol and erythritol. In addition, the flavoring agents can be natural flavoring agent such as thaumatin, stevia extract (rebaudioside A, glycyrrhizin and the like); or synthetic flavoring agent such as saccharin, aspartame and the like. When the food composition is prepared as a beverage, the natural carbohydrate can be included as 1-20 g per 100 mL of the composition in general, and preferably 5-12 g per 100 mL of the composition.

The food composition of the present disclosure can be prepared as a form of powder, granule, tablet, capsule or beverage, and used as foods, beverage, gum, tea, vitamin complex and health supplement foods.

Also, the food composition of the present disclosure can be added to medicaments, foods and beverages to improve concentration ability, or to prevent or ameliorate cognitive impairment. For instance, the food composition of the present disclosure can be added to foods, beverages, gums, teas, vitamin complexes, health supplement foods and the like.

The food composition of the present disclosure can be added to food or beverage to improve concentration ability, or to prevent or ameliorate cognitive impairment. The food composition of the present disclosure can be included as 1-5 wt% based on total weight of the food. The same can be included in the ratio of 0.02-10 g in 100 mL of the beverage, and preferably 0.3-1 g.

[Advantageous Effect]

The pharmaceutical composition of the present disclosure comprising Eclalbasaponin or derivative thereof, or the *Eclipta prostrata* extract containing the same as an active ingredient can effectively prevent or treat cognitive impairment or concentration impairment.

Also, the food composition of the present disclosure comprising Eclalbasaponin or derivative thereof, or the *Eclipta prostrata* extract containing the same as an active ingredient can effectively prevent or ameliorate cognitive impairment or concentration impairment.

[Brief Description of Drawings]

Figure 1 is a graph showing improving effect of the Eclalbasaponin II of the present disclosure on memory and learning abilities.

Figure 2 is a graph showing improving effect of the Eclalbasaponin I of the present disclosure on memory and learning abilities.

Figure 3 is a graph showing improving effect of the 70% ethanol extract of *Eclipta prostrata* of the present disclosure on memory and learning abilities.

Figure 4 is a graph showing improving effect of the 30% ethanol extract of *Eclipta prostrata* of the present disclosure on memory and learning abilities.

Figure 5 is a graph showing improving effect of the Eclalbasaponin II of the present disclosure on concentration ability.

[Description of Embodiments]

The present disclosure will be described more fully hereinafter with reference to the accompanying examples. However, the present disclosure may be embodied in many different forms, and should not be construed as being limited to the embodiments set forth herein.

In addition, reagents and solvents used herein were purchased from Sigma unless otherwise said, and optical rotatory power was measured by using the JASCO P-1020 polarimeter. UV was measured by using the Hitachi JP/U3010, IR was measured by using the JASCO FT/IR-5300, NMR was measured by using the Avance 400 (400 MHz) of Bruker and FAB Mass spectrum was measured by using the JEOL JMS-700 mass spectrometer.

**Preparation Example 1: Preparation of the 70% ethanol extract of *Eclipta prostrata* containing Eclalbasaponin**

Whole plant of dried *Eclipta prostrata* (5 kg) was purchased (Gyeongdong Herbal Market, Seoul, Republic of Korea) and powdered with a domestic mixer. A 10-fold (v/v) amount of 70% ethanol (v/v) was added to the powder and the same was extracted twice with an ultrasonic extractor (POWERSONIC 420, Hwashin Tech) for 2 hours at 60 °C in water bath to yield the extract. The extract was filtered with absorbent cotton and concentrated under reduced pressure with a vacuum evaporator (N-1000, EYELA) to yield a 70% (v/v) soft extract. The soft extract was lyophilized with a freeze drier (FDU-2000, EYELA) to yield powder (yield: 17.8 %).

**Preparation Example 2: Preparation of the 30% ethanol extract of *Eclipta prostrata* containing Eclalbasaponin**

The *Eclipta prostrata* extract was yielded in the same manner as Preparation Example 1 except that 30% (v/v) ethanol was used as an extraction solvent (yield: 6.48%).

**Preparation Example 3: Separation and Purification of Eclalbasaponin**

The 70% ethanol extract of *Eclipta prostrata* (44.0 g) prepared in Preparation Example 1 was suspended in distilled water (500 mL) and sequentially fractioned with hexane (500 ml×3), ethyl acetate (500 ml×3) and butanol (500 ml×3) to obtain hexane fraction (2.86 g), ethyl acetate fraction (4.14 g), butanol fraction (6.07 g) and water fraction (30.9 g) respectively. The ethyl acetate fraction was divided into 13 small fractions (small fraction 1 to small fraction 13) by conducting column chromatography (silica gel, 4.6×40 cm, 70-230 mesh) by using $CH_2Cl_2$-MeOH mixed solvents [1:0, 19:1, 9:1, 4:1, 1:1, 0:1; 2000 mL respectively] as mobile phases. Eclalbasaponin I (150 mg) and eclalbasaponin II (121.0 mg) were separated by conducting Sephadex LH-20 column chromatography (Ø3.3×41 cm) on the small fraction 12 (570 mg) amongst 13 small fractions, by using MeOH as a mobile phase. Eclalbasaponin III, IV, V and VI were obtained through the above method. Structures of the separated compounds were defined through comparison between NMR data analysis and the references.

eclalbasaponin I: $^1$H-NMR (400 MHz pyridine-$d_5$) $\delta$: 0.74, 0.85, 0.85, 0.89, 0.98, 1.14, 1.70 (each 3H, s, $H_3$-23, 24, 25, 26, 27, 28, 29, 30), 3.28 (1H, dd, *J* = 4.4, 12 Hz, H-3), 3.91 (1H, m, Glc H-2), 4.80 (1H, d, *J* = 7.6 Hz, Glc H-1), 5.16 (1H, br s, H-16), 5.47 (1H, s, H-12), 6.19 (1H, d, *J* = 8.4 Hz, 28-*O*-Glc H-1).

eclalbasaponin II: $^1$H-NMR (400 MHz pyridine-$d_5$) $\delta$: 0.85, 0.98, 1.01, 1.05, 1.17, 1.29, 1.85 ($H_3$-23, 24, 25, 26, 27, 29, 30), 3.42 (1H, br d, *J* = 11.6 Hz, H-3), 4.04 (1H, m, Glc H-2), 4.94 (1H, d, J = 7.6 Hz, Glc H-1), 5.24 (1H, br s, H-16), 5.63 (1H, br s, H-12).

eclalbasaponin III: $^1$H-NMR (pyridine-$d_5$) $\delta$: 0.88, 1.00, 1.04, 1.09, 1.12, 1.25, 1.82 ($H_3$-23, 24, 25, 26, 27, 29, 30), 3.29 (1H, dd, *J* = 4.0, 11.7 Hz, H-3), 4.89 (1H, d, *J* = 7.7 Hz, Glc H-1), 5.30 (1H, br s, H-16), 5.36 (1H, d, *J* = 7.4 Hz, Glc' H-1), 5.61 (1H, br s, H-12), 6.30 (1H, d, *J* = 8.0 Hz, 28-*O*-Glc H-1) (Shoji *et al,* 1994).

eclalbasaponin IV: $^1$H-NMR (pyridine-$d_5$) $\delta$: 0.86, 1.03, 1.07, 1.10, 1.19, 1.27, 1.85 ($H_3$-23, 24, 25, 26, 27, 29, 30), 3.31 (1H, dd, *J* = 4.4, 11.7 Hz, H-3), 4.92 (1H, d, *J* = 7.3 Hz, Glc H-1), 5.25 (1H, br s, H-16), 5.37 (1H, d, *J* = 7.7 Hz, Glc' H-1), 5.65 (1H, br s, H-12) (Shoji *et al,* 1994)

eclalbasaponin V: $^1$H-NMR (pyridine-$d_5$) $\delta$: 0.80, 0.99, 1.06, 1.17, 1.18, 1.43, 1.84 ($H_3$-23, 24, 25, 26, 27, 29, 30), 3.34 (1H, dd, *J* = 4.0, 11.3 Hz, H-3), 4.99 (1H, d, *J* = 7.7 Hz, Glc H-1), 5.24 (1H, br s, H-16), 5.63 (1H, br s, H-12) (Shoji *et al,* 1994)

eclalbasaponin VI: $^1$H-NMR (pyridine-$d_5$) $\delta$: 0.83, 1.01, 1.04, 1.10, 1.17, 1.43, 1.83 ($H_3$-23, 24, 25, 26, 27, 29, 30), 3.35 (1H, br d, *J* = 11.0 Hz, H-3), 4.99 (1H, d, *J* = 7.7 Hz, Glc H-1), 5.31 (1H, br s, H-16), 5.60 (1H, br s, H-12). 6.32 (1H, d, *J* = 8.1 Hz, 28-*O*-Glc H-1) (Shoji *et al,* 1994)

$^{13}$C NMR spectral data (100 MHz pyridine-$d_5$)

| C | eclalbasaponin I | eclalbasaponin II |
|---|---|---|
| 1 | 38.9 | 39.3 |
| 2 | 26.7 | 27.1 |
| 3 | 88.9 | 89.3 |
| 4 | 39.6 | 40.0 |
| 5 | 56.0 | 56.4 |
| 6 | 18.6 | 19.0 |
| 7 | 33.5 | 34.0 |
| 8 | 40.2 | 40.4 |
| 9 | 47.2 | 47.6 |
| 10 | 37.1 | 37.5 |
| 11 | 23.9 | 24.3 |
| 12 | 122.8 | 122.8 |
| 13 | 144.5 | 145.6 |
| 14 | 42.1 | 41.9 |
| 15 | 36.2 | 36.7 |
| 16 | 74.5 | 75.2 |
| 17 | 49.2 | 49.4 |
| 18 | 41.4 | 42.6 |
| 19 | 47.2 | 47.8 |
| 20 | 30.9 | 31.5 |
| 21 | 36.0 | 36.6 |
| 22 | 32.3 | 33.3 |
| 23 | 28.3 | 28.7 |
| 24 | 17.1 | 17.5 |
| 25 | 15.7 | 16.1 |
| 26 | 17.6 | 18.0 |
| 27 | 27.3 | 27.7 |
| 28 | 176.0 | 180.5 |
| 29 | 33.3 | 33.8 |
| 30 | 24.6 | 25.2 |
| 3-*O*-Glc | | |
| 1 | 106.9 | 107.3 |
| 2 | 75.8 | 76.3 |
| 3 | 79.4 | 79.2 |
| 4 | 71.9 | 72.3 |
| 5 | 78.8 | 78.8 |
| 6 | 63.1 | 63.5 |

(continued)

| 28-*O*-Glc | | |
|---|---|---|
| 1 | 95.9 | |
| 2 | 74.2 | |
| 3 | 78.9 | |
| 4 | 71.1 | |
| 5 | 78.3 | |
| 6 | 62.2 | |

**Examples: Observation of Treating Effect on Cognitive Impairment (Dementia)**

To observe treating effect of the pharmaceutical composition of the present disclosure comprising Eclalbasaponin or derivative thereof, or the *Eclipta prostrata* extract containing the same on cognitive impairment, especially dementia, an experiment using an animal model of memory impairment induced by scopolamine was conducted. Detailed method of the experiment follows.

1) Preparation of laboratory animals

6-week old ICR mice in about 26 g to 28 g (Orientbio Inc, Republic of Korea) were received water and feed without constraint and adapted for 5 days under an environment having about 23±2°C of temperature, about 60±10% of humidity and 12-hour light/dark cycle (animal laboratory at College of Pharmacy, Kyung Hee University), and then used for the experiment.

2) Statistics process

Every experiment result was processed by using ANOVA (one-way analysis of variance) and a significance test was conducted at a level of $p < 0.05$ or below by using Student-Newman-Keuls Test when significance was recognized as exists.

3) Example 1: Passive avoidance test 1

A passive avoidance apparatus was prepared for the experiment. The apparatus has 2 separated chambers (the first chamber and the second chamber) and there is a guillotine-shaped passage that connects the first chamber and the second chamber. The first chamber was maintained brightly by using a light, and the second chamber was maintained darkly. A grid was installed on the floor of the second chamber. The grid generates 0.5 mA of electric shock for 3 seconds when the laboratory animal moved into the dark chamber.

The mice prepared in the above 1) were separated into 7 groups (10 mice per each group) comprised of drug administration groups 1 to 4 and controls 1 to 3. The Eclalbasaponin II prepared in Preparation Example 3 was dissolved in 10% Tween 80 (Polyoxyethylene sorbitan monooleate: Sigma, U.S.A.), and administered to the drug administration groups 1-4 in an amount of 2.5, 5, 10 and 20 mg/Kg respectively. Meanwhile, 5 mg/Kg of dopenezil was administered to the control 3, and 0.15 mL of 10% Tween 80 was administered to the controls 1 and 2 respectively.

About an hour later, 1 mg/Kg of scopolamine, which was dissolved in distilled water, was intraperitoneally administered to the drug administration groups 1-4 and controls 2-3 (Ebert, U. et al., Eur. J. Clin. Invest., 28, pp944-949, 1998), and 0.9% of saline solution was intraperitoneally administered to the control 1. After 30 minutes, passive avoidance learning was conducted on the drug administration groups 1-4 and controls 1-3. In detail, the mice were located in the first chamber (*i.e.* bright space) and the guillotine-shaped passage was open after about 10 seconds of observation time, and then latency time of the mice moving to the second chamber (*i.e.* dark space) was measured. The mice that did not move to the second chamber (*i.e.* dark space) after 60 seconds from opening of the guillotine-shaped passage were excluded.

24 hours after the passive avoidance learning, a main experiment was conducted on the drug administration groups 1-4 and controls 1-3 respectively. Latency time of the mice of each group moving their 4 legs into the dark space after 10 seconds of observation time from opening of the guillotine-shaped passage was measured up to 300 seconds. It means that the passive avoidance learning and memory are better if the latency time is longer.

Average latency time of the mice of each group at the passive avoidance learning and the main experiment

were shown in Table 2 and Figure 1.

[Table 2]

| Group | Passive Avoidance Learning (sec.) | Main Experiment (sec.) |
|---|---|---|
| Drug Admin. Gr. 1(2.5 mg/Kg) | 26.44±4.217 | 146.00±34.950 |
| Drug Admin. Gr. 2(5 mg/Kg) | 29.11±2.831 | 196.90±24.750 |
| Drug Admin. Gr. 3(10 mg/Kg) | 30.67±4.460 | 159.90±30.410 |
| Drug Admin. Gr. 4(20 mg/Kg) | 30.56±4.154 | 91.00±11.970 |
| Control 1 (Control) | 30.83±4.806 | 276.70±17.300 |
| Control 2 (Scopolamine) | 30.44±4.580 | 33.00±7.464 |
| Control 3 (Donepezil) | 37.67±4.726 | 185.80±21.420 |

As shown in Table 2 and Figure 1, latency time of moving to the second chamber of the drug administration groups 1-3, wherein Eclalbasaponin II prepared in Preparation Example 3 was administered, were remarkably extended compared to that of the control 2, wherein scopolamine was administered. In detail, latency time of the drug administration group 1, wherein 2.5 mg/Kg of Eclalbasaponin II was administered, was about 4.4-fold extended compared to that of the control 2; latency time of the drug administration group 2, wherein 5 mg/Kg of Eclalbasaponin II was administered, was about 5.9-fold extended compared to that of the control 2; and latency time of the drug administration group 3, wherein 10 mg/Kg of Eclalbasaponin II was administered was about 4.8-fold extended compared to that of the control 2. Specifically, it was observed that in the drug administration groups 2 and 3, memory and learning abilities of the memory-impaired mouse due to administration of scopolamine were improved and this improvement was almost similar to that of the control 3 wherein dopenezil was administered. Accordingly, it was confirmed that Eclalbasaponin II was effective for preventing or treating cognitive impairment such as dementia.

4) Example 2 : Passive avoidance test 2

Eclalbasaponin I prepared through the method explained in Preparation Example 3 was dissolved in 10% Tween 80, and administered to the drug administration groups 1-4 in amounts of 2.5 mg/kg, 5 mg/kg, 10 mg/kg and 20 mg/kg respectively. The passive avoidance test was conducted on the drug administration groups 1-4 and the controls 1-3 in the same manner as Example 1.

Test result therefrom was shown in Table 3 and Figure 2.

[Table 3]

| Group | Passive Avoidance Learning (sec.) | Main Experiment (sec.) |
|---|---|---|
| Drug Admin. Gr. 1(2.5 mg/Kg) | 30.11±6.775 | 74.88±14.950 |
| Drug Admin. Gr. 2 (5 mg/Kg) | 35.44±7.211 | 99.00±13.910 |
| Drug Admin. Gr. 3 (10 mg/Kg) | 31.22±5.351 | 144.30±25.680 |
| Drug Admin. Gr. 4 (20 mg/Kg) | 21.22±2.808 | 127.40±26.200 |
| Control 1 (Control) | 23.00±6.608 | 286.00±8.324 |
| Control 2 (Scopolamine) | 28.11±4.979 | 56.67±11.360 |
| Control 3 (Donepezil) | 32.67±7.022 | 197.40±28.500 |

As shown in Table 3 and Figure 2, latency time of moving to the second chamber of the drug administration groups 1-4, wherein Eclalbasaponin I prepared in Preparation Example 3 was administered, were remarkably extended compared to that of the control 2, wherein scopolamine was administered. In detail, latency time of the drug administration group 3, wherein 10 mg/Kg of Eclalbasaponin I was administered, was about 2.54-fold extended compared to that of the control 2.

Accordingly, it was confirmed that Eclalbasaponin I was effective for preventing or treating cognitive impairment such as dementia.

5) Example 3: Passive avoidance test 3

*Eclipta prostrata* extract extracted with 70% ethanol prepared in Preparation Example 1 was dissolved in 10% Tween 80, and administered to the drug administration groups 1-4 in amounts of 25 mg/kg, 50 mg/kg, 100 mg/kg and 200 mg/kg respectively. The passive avoidance test was conducted on the drug administration groups 1-4 and the controls 1-3 in the same manner as Example 1.

Test result therefrom was shown in Table 4 and Figure 3.

[Table 4]

| Group | Passive Avoidance Learning (sec.) | Main Experiment (sec.) |
|---|---|---|
| Drug Admin. Gr. 1 (25 mg/Kg) | 30.89±4.550 | 91.67±17.320 |
| Drug Admin. Gr. 2 (50 mg/Kg) | 32.33±4.983 | 118.70±23.240 |
| Drug Admin. Gr. 3 (100 mg/Kg) | 40.33±6.791. | 151.10±32.460 |
| Drug Admin. Gr. 4 (200 mg/Kg) | 23.22±4.858 | 101.30±24.750 |
| Control 1 (Control) | 27.83±5.974 | 277.30±19.290 |
| Control 2 (Scopolamine) | 29.67±4.265 | 30.63±6.803 |
| Control 3 (Donepezil) | 32.67±5.593 | 187.80±23.850 |

As shown in Table 4 and Figure 3, latency time of moving to the second chamber of the drug administration groups 1-4, wherein 70% ethanol extract of *Eclipta prostrata* containing Eclalbasaponin prepared in Preparation Example 1 was administered, were remarkably extended compared to that of the control 2, wherein scopolamine was administered. In detail, latency time of the drug administration group 2, wherein 50 mg/Kg of 70% ethanol extract of *Eclipta prostrata* was administered, was about 3.8-fold extended compared to that of the control 2; and latency time of the drug administration group 3, wherein 100 mg/Kg of 70% ethanol extract of *Eclipta prostrata* was administered, was about 4.9-fold extended compared to that of the control 2.

Accordingly, it was confirmed that 70% ethanol extract of *Eclipta prostrata* containing Eclalbasaponin prepared in Preparation Example 1 was effective for preventing or treating cognitive impairment such as dementia.

6) Example 4: Passive avoidance test 4

*Eclipta prostrata* extract extracted with 30% ethanol prepared in Preparation Example 2 was dissolved in 10% Tween 80, and administered to the drug administration groups 1-4 in amounts of 25 mg/kg, 50 mg/kg, 100 mg/kg and 200 mg/kg respectively. The passive avoidance test was conducted on the drug administration groups 1-4 and the controls 1-3 in the same manner as Example 1.

Test result therefrom was shown in Table 5 and Figure 4.

[Table 5]

| Group | Passive Avoidance Learning (sec.) | Main Experiment (sec.) |
|---|---|---|
| Drug Admin. Gr. 1 (25 mg/Kg) | 36.00±5.382 | 82.25±20.00 |
| Drug Admin. Gr. 2 (50 mg/Kg) | 36.67±6.249 | 140.60±20.10 |
| Drug Admin. Gr. 3 (100 mg/Kg) | 35.89±4.823 | 112.10±17.14 |
| Drug Admin. Gr. 4 (200 mg/Kg) | 34.00±5.323 | 97.50±12.96 |
| Control 1 (Control) | 21.33±2.333 | 265.80±21.08 |
| Control 2 (Scopolamine) | 38.78±6.300 | 55.14±13.94 |
| Control 3 (Donepezil) | 36.00±6.640 | 194.80±30.01 |

As shown in Table 5 and Figure 4, latency time of moving to the second chamber of the drug administration group 2, wherein 30% ethanol extract of *Eclipta prostrata* containing Eclalbasaponin prepared in Preparation Example 2 was administered, were remarkably extended compared to that of the control 2, wherein scopolamine was administered. In detail, latency time of the drug administration group 2, wherein 50 mg/Kg of 30% ethanol extract of *Eclipta prostrata*

was administered, was about 2.5-fold extended compared to that of the control 2.

Accordingly, it was confirmed that 30% ethanol extract of *Eclipta prostrata* containing Eclalbasaponin prepared in Preparation Example 2 was effective for preventing or treating cognitive impairment such as dementia.

7) Example 5: Y-maze test

A Y-maze was prepared for the experiment. The Y-maze has 3 branches and each branch has 42 cm of length, 3 cm of width and 12 cm of height. Angle between any two arms is 120 degrees and the arms were made of black polyvinyl resin.

The mice prepared in the above 1) were separated into 7 groups (8-10 mice per each group) comprised of drug administration groups 1 to 4 and controls 1 to 3.

The Eclalbasaponin II prepared in Preparation Example 3 (20 mg) was dissolved in 5 mL of 10% Tween 80 (Polyoxyethylene sorbitan monooleate: Sigma, U.S.A.), and administered to the drug administration groups 1-4 in an amounts of 2.5, 5, 10 and 20 mg/Kg respectively. Meanwhile, 5 mg/Kg of dopenezil was administered to the control 3, and 0.15 mL of 10% Tween 80 was administered to the controls 2 and 3 respectively.

About 30 minutes later, 1 mg/Kg of scopolamine, which was dissolved in distilled water, was intraperitoneally administered to the drug administration groups 1-4 and controls 2-3 (Ebert, U. et al., Eur. J. Clin. Invest., 28, pp. 944-949, 1998), and 0.9% of saline solution was intraperitoneally administered to the control 1.

After 30 minutes, mice of the drug administration groups 1-4 and the controls 1-3 were carefully located on each branches of the Y-maze, which was divided as A, B and C respectively, and let the mice to move freely for 8 minutes, and then recorded branches that mice have entered. The entrance was recorded when the entire body (*i.e.* including the tail) has completely entered. It was also recorded when the mice entered again into the branch that the mice have entered before.

1 point was scored when the mouse sequentially entered into 3 different branches (actual alternation). Alternation behavior is defined as sequentially entering into every 3 branch, and it was converted into % *via* Equation 1 below (Sarter, M. et al., Psychopharmacology., 94, pp. 491-495, 1998).

[Equation 1]

Alternation Behavior (%) = (Actual Alternation / Maximum Alternation) s 100

(Maximum Alternation: Total Entrance - 2)

Converted alternation behavior by Equation 1 was shown in Table 5 and Figure 6.

[Table 6]

| Group | Alternation Behavior (%) |
|---|---|
| Drug Admin. Gr. 1 (2.5 mg/Kg) | 53.89±2.690 |
| Drug Admin. Gr. 2 (5 mg/Kg) | 57.38±2.104 |
| Drug Admin. Gr. 3 (10 mg/Kg) | 62.50±2.283 |
| Drug Admin. Gr. 4 (20mg/Kg) | 58.00±3.586 |
| Control 1 (Control) | 75.89±2.653 |
| Control 2 (Scopolamine) | 50.20±1.988 |
| Control 3 (Donepezil) | 64.33±1.667 |

As shown in Table 6 and Figure 5, alternation behavior of the drug administration group 3, wherein Eclalbasaponin II prepared in Preparation Example 3 was administered, was increased compared to that of the control 2, wherein only scopolamine was administered, and it was similar to that of the control 3, wherein donepezil was administered.

Accordingly, it was confirmed that Eclalbasaponin II prepared in Preparation Example 3 was effective in preventing or treating cognitive impairment such as dementia and in improving concentration ability.

In addition, a death was not observed within 2 weeks after a single administration of 2 g/kg of Eclalbasaponin, or *Eclipta prostrata* extract containing the same.

[Industrial Applicability]

The composition of the present disclosure comprising Eclalbasaponin or derivative thereof, or the *Eclipta prostrata* extract containing the same as an active ingredient can effectively prevent, ameliorate or treat cognitive impairment or concentration impairment.

## Claims

1. A pharmaceutical composition for use in preventing or treating cognitive impairment or concentration impairment, comprising Eclalbasaponin or derivative thereof, represented by Formula 1 below, as an active ingredient:

[Formula 1]

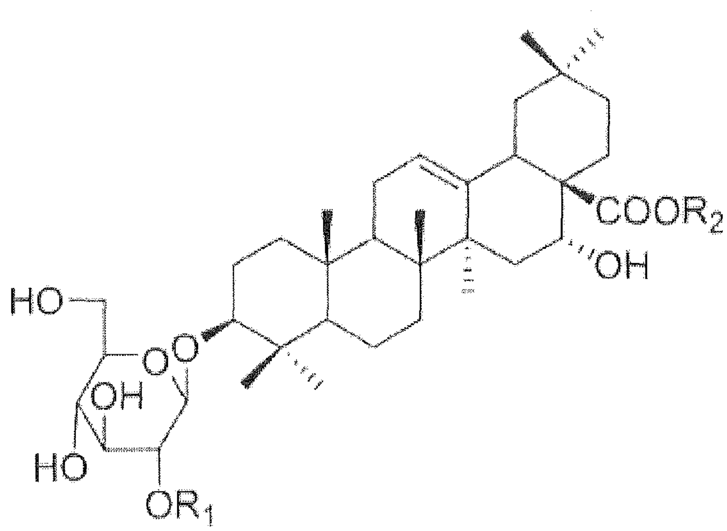

in Formula 1,

$R_1$ is H, $SO_3H$ or glucose (Glc), and
$R_2$ is H or glucose (Glc).

2. The pharmaceutical composition for use according to claim 1, wherein the Eclalbasaponin or derivative thereof is a compound stated in Table 1 below.

[Table 1]

| Compound | $R_1$ | $R_2$ |
|---|---|---|
| Eclalbasaponin I | H | Glc |
| Eclalbasaponin II | H | H |
| Eclalbasaponin III | Glc | Glc |
| Eclalbasaponin IV | Glc | H |
| Eclalbasaponin V | $SO_3H$ | H |
| Eclalbasaponin VI | $SO_3H$ | Glc |

**3.** The pharmaceutical composition for use according to claim 2, wherein the Eclalbasaponin or derivative thereof is Eclalbasaponin II.

**4.** The pharmaceutical composition for use according to claim 1, wherein the Eclalbasaponin or derivative thereof is yielded from *Eclipta prostrata* extract.

**5.** The pharmaceutical composition for use according to claim 1, wherein the cognitive impairment is delirium, dementia or amnesia.

**6.** The pharmaceutical composition for use according to claim 5, wherein the dementia is Alzheimer's dementia or vascular dementia.

**7.** A pharmaceutical composition for use in preventing or treating cognitive impairment or concentration impairment, comprising *Eclipta prostrata* extract containing Eclalbasaponin or derivative thereof, represented by Formula 1 below:

[Formula 1]

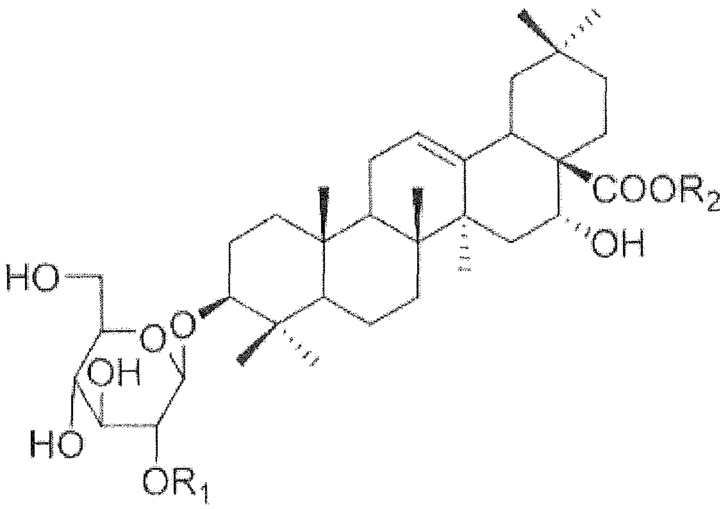

in Formula 1,

$R_1$ is H, $SO_3H$ or glucose (Glc), and
$R_2$ is H or glucose (Glc).

**8.** The pharmaceutical composition for use according to claim 7, wherein the cognitive impairment is delirium, dementia or amnesia.

**9.** The pharmaceutical composition for use according to claim 8, wherein the dementia is Alzheimer's dementia or vascular dementia.

**10.** A food composition for use in preventing or ameliorating cognitive impairment or concentration impairment, comprising Eclalbasaponin or derivative thereof, represented by Formula 1 below:

[Formula 1]

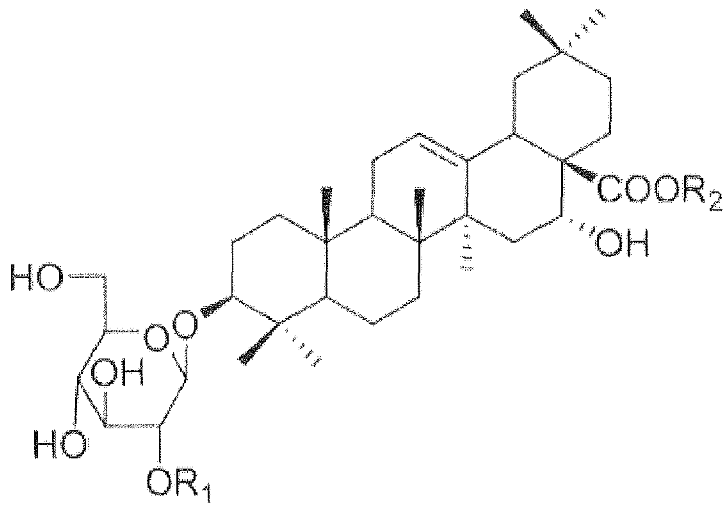

in Formula 1,

$R_1$ is H, $SO_3H$ or glucose (Glc), and
$R_2$ is H or glucose (Glc).

11. A food composition for use in preventing or ameliorating cognitive impairment or concentration impairment, comprising *Eclipta prostrata* extract containing Eclalbasaponin or derivative thereof, represented by Formula 1 below:

[Formula 1]

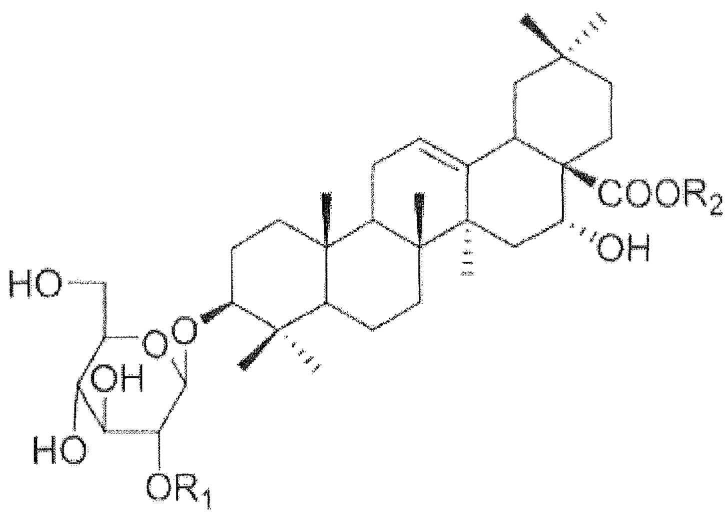

in Formula 1,

$R_1$ is H, $SO_3H$ or glucose (Glc), and
$R_2$ is H or glucose (Glc).

**Patentansprüche**

1. Pharmazeutische Zusammensetzung zur Verwendung in der Vorbeugung oder Behandlung von kongnitiver Beeinträchtigung oder Konzentrationsbeeinträchtigung, umfassend Eclalbasaponin oder ein Derivat davon gemäß Formel 1 unten als Wirkstoff:

[Formel 1]

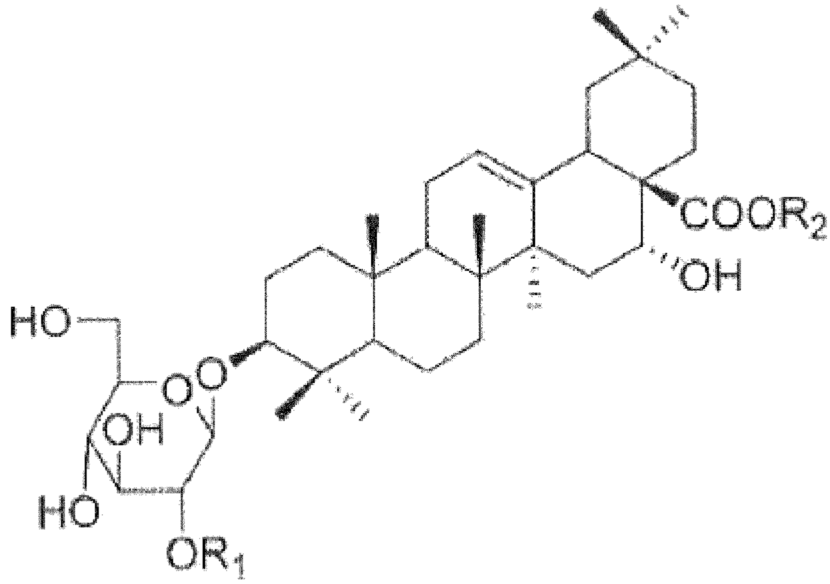

in Formel 1

bedeutet $R_1$ H, $SO_3H$ oder Glucose (Glc), und
bedeutet $R_2$ H oder Glucose (Glc).

2. Pharmazeutische Zusammensetzung zur Verwendung nach Anspruch 1, wobei es sich bei dem Eclalbasaponin oder Derivat davon um eine Verbindung gemäß Tabelle 1 unten handelt.

[Tabelle 1]

| Verbindung | $R_1$ | $R_2$ |
|---|---|---|
| Eclalbasaponin I | H | Glc |
| Eclalbasaponin II | H | H |
| Eclalbasaponin III | Glc | Glc |
| Eclalbasaponin IV | Glc | H |
| Eclalbasaponin V | $SO_3H$ | H |
| Eclalbasaponin VI | $SO_3H$ | Glc |

3. Pharmazeutische Zusammensetzung zur Verwendung nach Anspruch 2, wobei es sich bei dem Eclalbasaponin oder Derivat davon um Eclalbasaponin II handelt.

4. Pharmazeutische Zusammensetzung zur Verwendung nach Anspruch 1, wobei das Eclalbasaponin oder Derivat aus einem *Eclipta-prostrata*-Extrakt stammt.

5. Pharmazeutische Zusammensetzung zur Verwendung nach Anspruch 1, wobei es sich bei der kognitiven Beeinträchtigung um Delirium, Demenz oder Amnesie handelt.

6. Pharmazeutische Zusammensetzung zur Verwendung nach Anspruch 5, wobei es sich bei der Demenz um Alzheimer-Demenz oder vaskuläre Demenz handelt.

7. Pharmazeutische Zusammensetzung zur Verwendung in der Vorbeugung oder Behandlung von kongnitiver Beeinträchtigung oder Konzentrationsbeeinträchtigung, umfassend *Eclipta-prostrata*-Extrakt enthaltend Eclalbasaponin

oder ein Derivat davon gemäß Formel 1 unten als Wirkstoff:

[Formel 1]

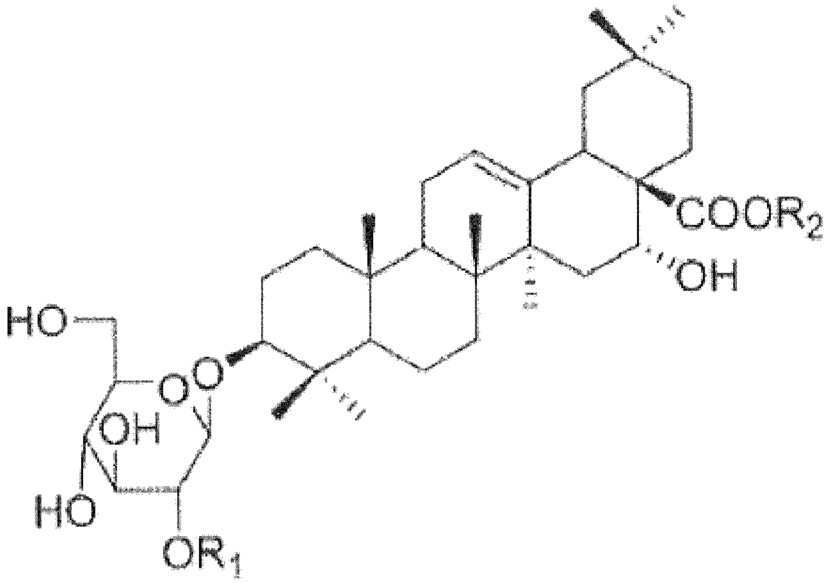

in Formel 1

bedeutet $R_1$ H, $SO_3H$ oder Glucose (Glc), und
bedeutet $R_2$ H oder Glucose (Glc).

**8.** Pharmazeutische Zusammensetzung zur Verwendung nach Anspruch 7, wobei es sich bei der kognitiven Beeinträchtigung um Delierium, Demenz oder Amnesie handelt.

**9.** Pharmazeutische Zusammensetzung zur Verwendung nach Anspruch 8, wobei es sich bei der Demenz um Alzheimer-Demenz oder vaskuläre Demenz handelt.

**10.** Nahrungsmittelzusammensetzung zur Verwendung in der Vorbeugung oder Linderung von kongnitiver Beeinträchtigung oder Konzentrationsbeeinträchtigung, umfassend Eclalbasaponin oder ein Derivat davon gemäß Formel 1 unten als Wirkstoff:

[Formel 1]

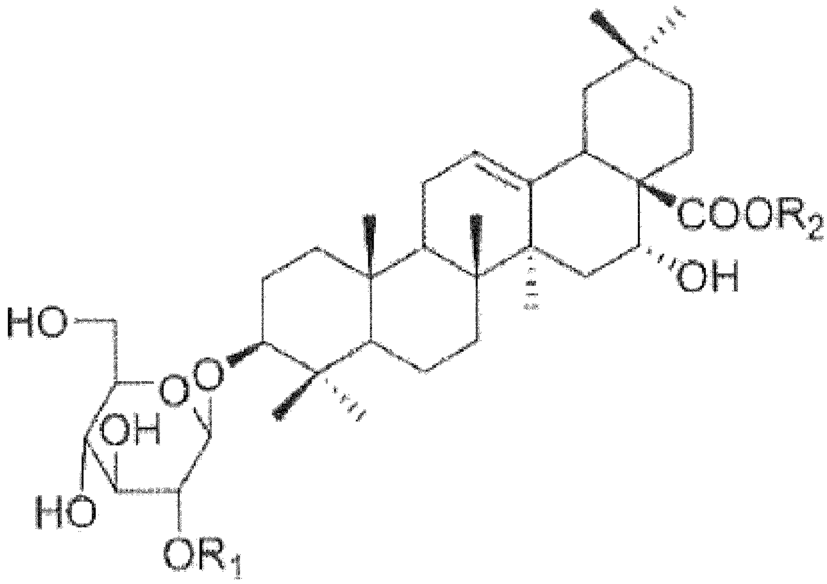

in Formel 1

bedeutet $R_1$ H, $SO_3H$ oder Glucose (Glc), und
bedeutet $R_2$ H oder Glucose (Glc).

11. Nahrungsmittelzusammensetzung zur Verwendung in der Vorbeugung oder Linderung von kongnitiver Beeinträchtigung oder Konzentrationsbeeinträchtigung, umfassend *Eclipta-prostrata*-Extrakt enthaltend Eclalbasaponin oder ein Derivat davon gemäß Formel 1 unten als Wirkstoff:

[Formel 1]

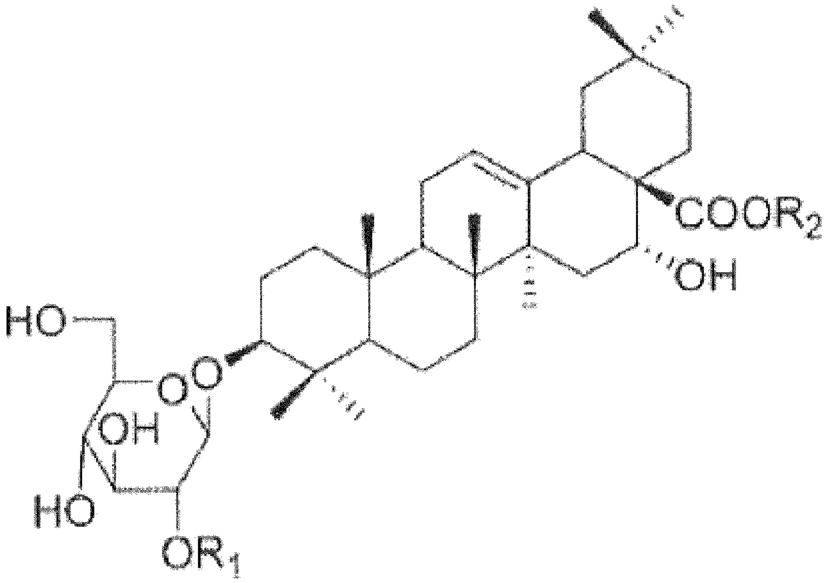

in Formel 1

bedeutet $R_1$ H, $SO_3H$ oder Glucose (Glc), und
bedeutet $R_2$ H oder Glucose (Glc).

## Revendications

1. Composition pharmaceutique pour utilisation dans la prévention ou le traitement d'un trouble cognitif ou d'un trouble de la concentration, comprenant de l'éclalbasaponine ou un dérivé de celle-ci, représentée par la formule 1 ci-dessous, en tant que substance active :

[Formule 1]

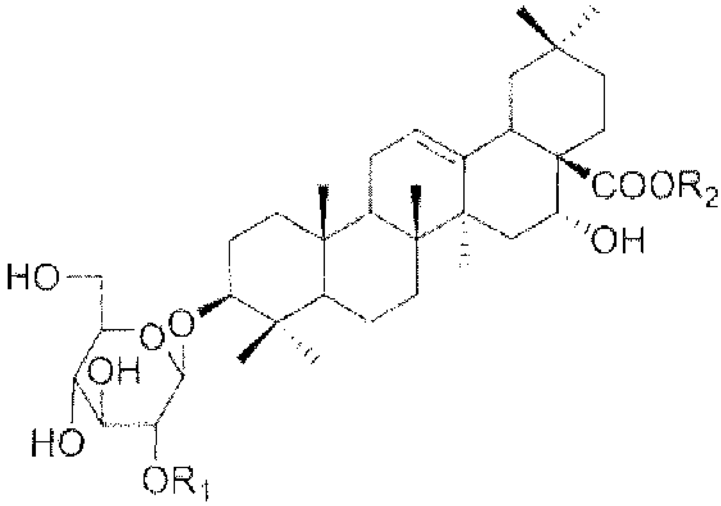

dans la formule 1,

$R_1$ est H, $SO_3H$ ou glucose (Glc), et
$R_2$ est H ou glucose (Glc).

2. Composition pharmaceutique pour utilisation selon la revendication 1, dans laquelle l'éclalbasaponine ou un dérivé de celle-ci est un composé décrit dans le tableau 1 ci-dessous.

[Tableau 1]

| Composé | $R_1$ | $R_2$ |
|---|---|---|
| Éclalbasaponine I | H | Glc |
| Éclalbasaponine II | H | H |
| Éclalbasaponine III | Glc | Glc |
| Éclalbasaponine IV | Glc | H |
| Éclalbasaponine V | $SO_3H$ | H |
| Éclalbasaponine VI | $SO_3H$ | Glc |

3. Composition pharmaceutique pour utilisation selon la revendication 2, dans laquelle l'éclalbasaponine ou un dérivé de celle-ci est l'éclalbasaponine II.

4. Composition pharmaceutique pour utilisation selon la revendication 1, dans laquelle l'éclalbasaponine ou un dérivé de celle-ci est obtenue à partir d'un extrait *d'Eclipta prostrata.*

5. Composition pharmaceutique pour utilisation selon la revendication 1, dans laquelle le trouble cognitif est un délire, une démence ou une amnésie.

6. Composition pharmaceutique pour utilisation selon la revendication 5, dans laquelle la démence est la démence d'Alzheimer ou la démence vasculaire.

**7.** Composition pharmaceutique pour utilisation dans la prévention ou le traitement d'un trouble cognitif ou d'un trouble de la concentration, comprenant un extrait d'*Eclipta prostrata* contenant de l'éclalbasaponine ou un dérivé de celle-ci, représentée par la formule 1 ci-dessous :

[Formule 1]

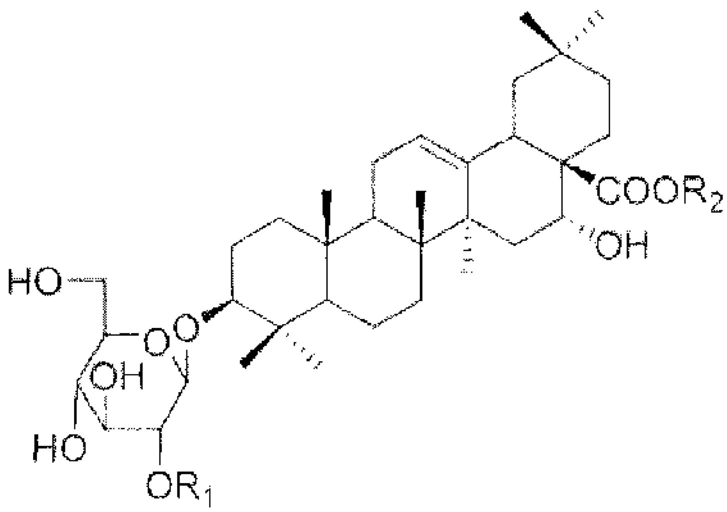

dans la formule 1,

$R_1$ est H, $SO_3H$ ou glucose (Glc), et
$R_2$ est H ou glucose (Glc).

**8.** Composition pharmaceutique pour utilisation selon la revendication 7, dans laquelle le trouble cognitif est un délire, une démence ou une amnésie.

**9.** Composition pharmaceutique pour utilisation selon la revendication 8, dans laquelle la démence est la démence d'Alzheimer ou la démence vasculaire.

**10.** Composition alimentaire pour utilisation dans la prévention ou l'amélioration d'un trouble cognitif ou d'un trouble de la concentration, comprenant de l'éclalbasaponine ou un dérivé de celle-ci, représentée par la formule 1 ci-dessous :

[Formule 1]

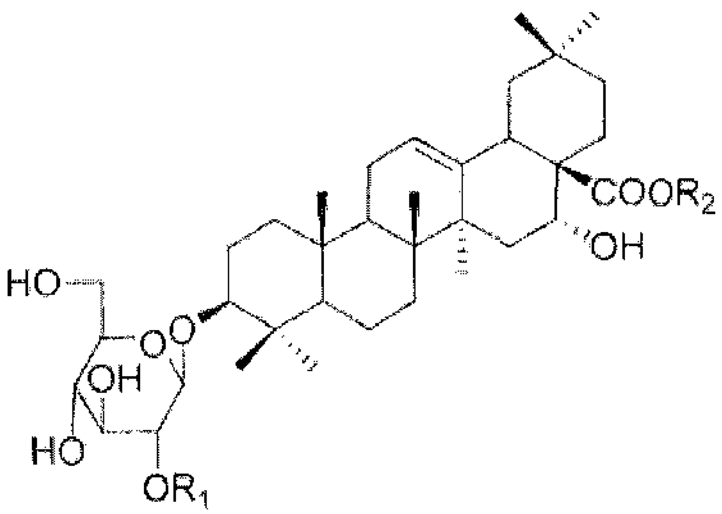

dans la formule 1,

$R_1$ est H, $SO_3H$ ou glucose (Glc), et
$R_2$ est H ou glucose (Glc).

**11.** Composition alimentaire pour utilisation dans la prévention ou l'amélioration d'un trouble cognitif ou d'un trouble de la concentration, comprenant un extrait d'*Eclipta prostrata* contenant de l'éclalbasaponine ou un dérivé de celle-ci, représentée par la formule 1 ci-dessous :

[Formule 1]

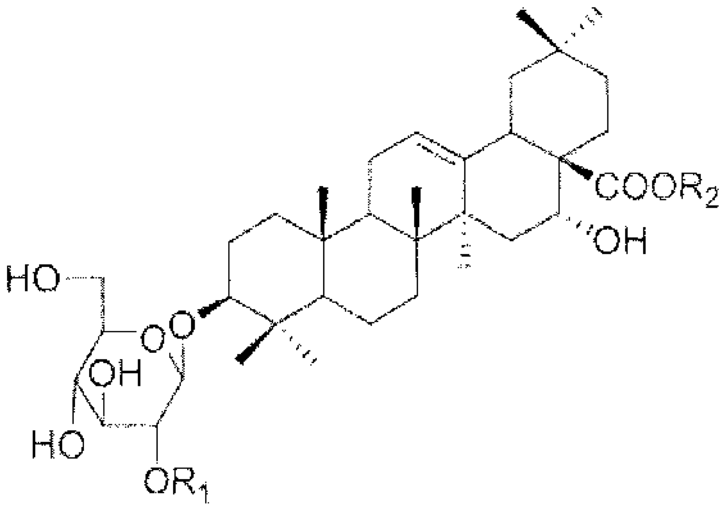

dans la formule 1,

$R_1$ est H, $SO_3H$ ou glucose (Glc), et
$R_2$ est H ou glucose (Glc).

【FIG.1】

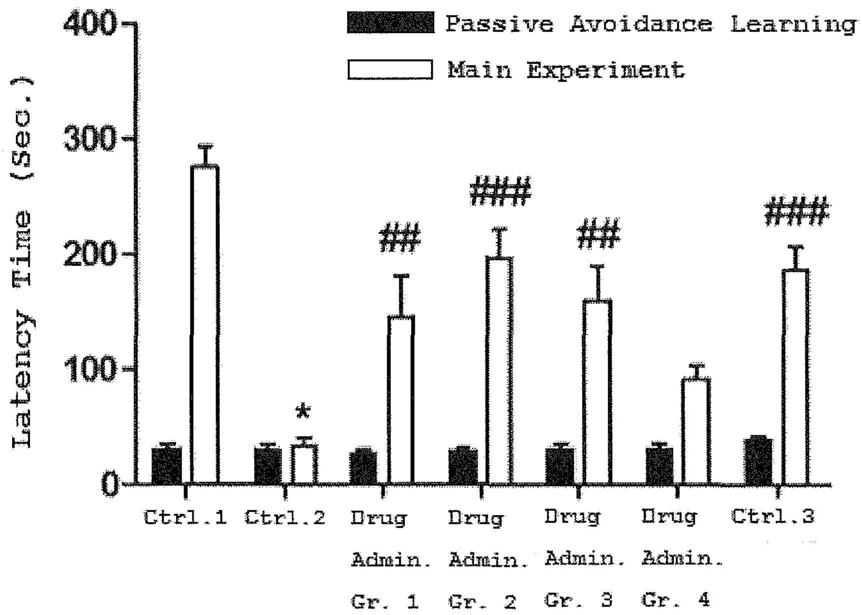
Passive Avoidance Learning
Main Experiment
Latency Time (Sec.)
400
300
200
100
0
*
##
###
##
###
Ctrl.1
Ctrl.2
Drug Admin. Gr. 1
Drug Admin. Gr. 2
Drug Admin. Gr. 3
Drug Admin. Gr. 4
Ctrl.3

【FIG.2】

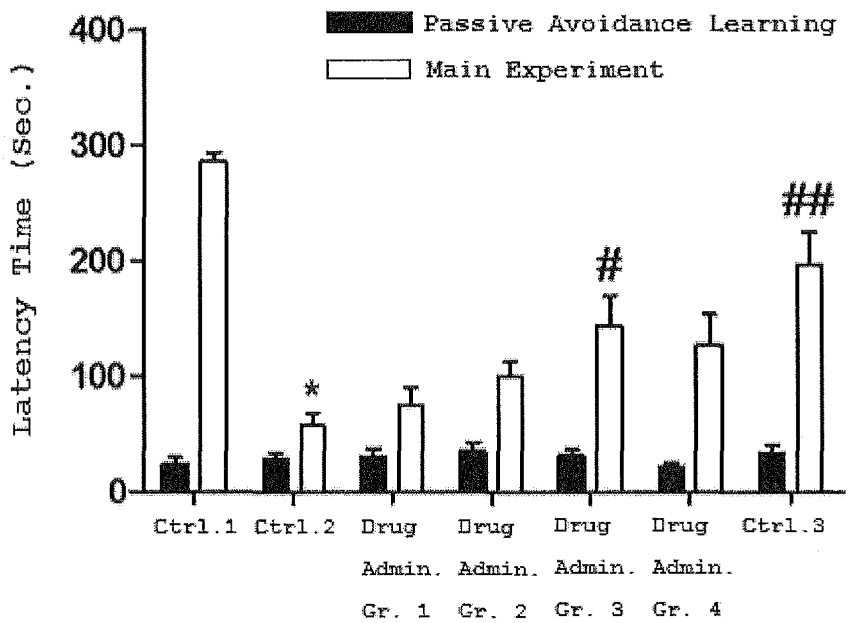
Passive Avoidance Learning
Main Experiment
Latency Time (Sec.)
400
300
200
100
0
*
#
##
Ctrl.1
Ctrl.2
Drug Admin. Gr. 1
Drug Admin. Gr. 2
Drug Admin. Gr. 3
Drug Admin. Gr. 4
Ctrl.3

【FIG.3】

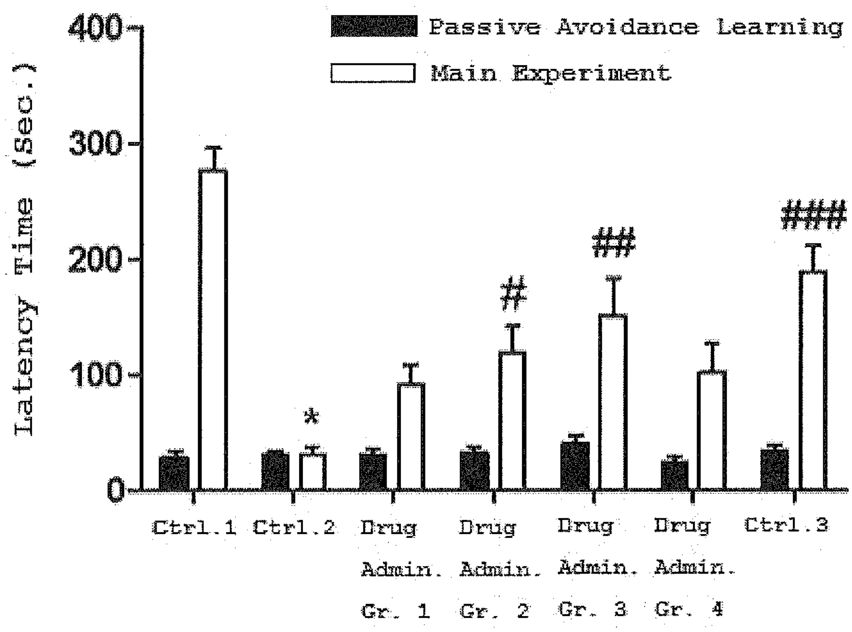
Passive Avoidance Learning
Main Experiment
Latency Time (Sec.)
400
300
200
100
0
*
#
##
###
Ctrl.1
Ctrl.2
Drug Admin. Gr. 1
Drug Admin. Gr. 2
Drug Admin. Gr. 3
Drug Admin. Gr. 4
Ctrl.3

【FIG.4】

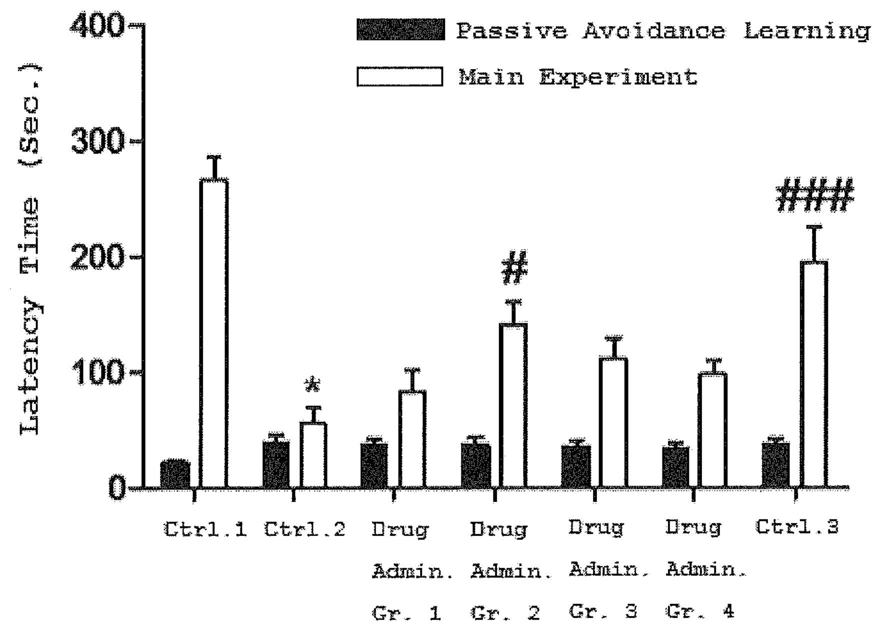
Passive Avoidance Learning
Main Experiment
Latency Time (Sec.)
400
300
200
100
0
*
#
###
Ctrl.1
Ctrl.2
Drug Admin. Gr. 1
Drug Admin. Gr. 2
Drug Admin. Gr. 3
Drug Admin. Gr. 4
Ctrl.3

【FIG.5】

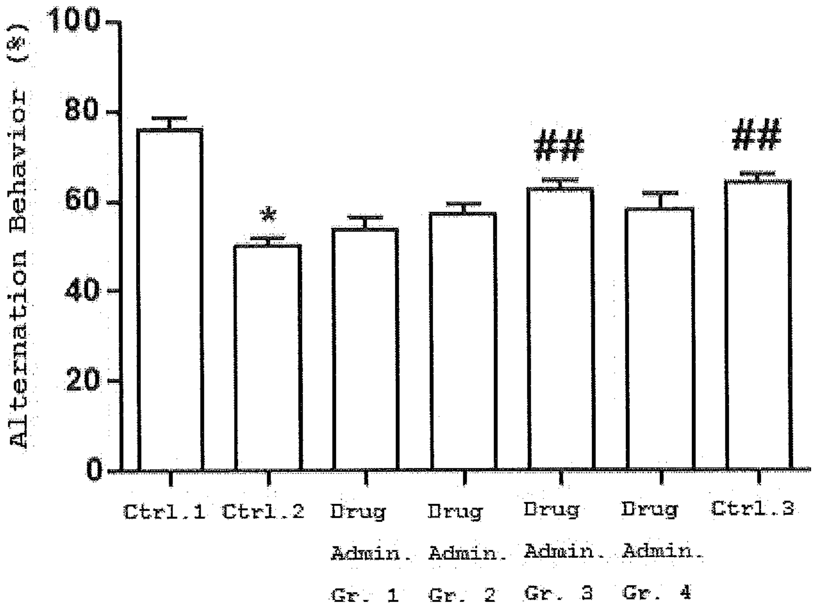
Alternation Behavior (%)
100
80
60
40
20
0
*
##
##
Ctrl.1
Ctrl.2
Drug Admin. Gr. 1
Drug Admin. Gr. 2
Drug Admin. Gr. 3
Drug Admin. Gr. 4
Ctrl.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004069182 A
- US 20120321694 A


### Non-patent literature cited in the description


- **SHOJI YAHARA et al.** *Chem. Pharm. Bull.,* 1994, vol. 42 (6), 1336-1338
- **YUKO NAKAHARA et al.** *Chem. Pharm. Bul.,* 2011, vol. 59 (11), 1329-1339
- **ERIC K. SHEA et al.** *J. Am. Chem. Soc.,* 2012, vol. 134, 13448-13457
- **EBERT, U. et al.** *Eur. J. Clin. Invest.,* 1998, vol. 28, 944-949
- **SARTER, M. et al.** *Psychopharmacology.,* 1998, vol. 94, 491-495